# EUROPEAN PATENT SPECIFICATION

(11) **EP 3 730 185 B1**
(45) Date of publication and mention of the grant of the patent: **08.09.2021**
(21) Application number: 19202659.9
(22) Date of filing: 11.10.2019
(51) Int. Cl.: A61N 1/05, A61N 1/372, A61N 1/375

(54) **IMPLANTABLE MEDICAL DEVICE COMPRISING A METAL/CERAMICS COMPOSITE HOUSING**
IMPLANTIERBARE MEDIZINISCHE VORRICHTUNG, METALL/KERAMIK-VERBUNDGEHÄUSE UMFASSEND
DISPOSITIF MÉDICAL IMPLANTABLE COMPRENANT UN BOÎTIER COMPOSITE EN MÉTAL/CÉRAMIQUE

(30) Priority: 26.04.2019 US 201916396388
(43) Date of publication of application: 28.10.2020
(73) Proprietor: Cairdac, 92160 Antony (FR)
(72) Inventor: REGNIER, Willy, 91160 Longjumeau (FR); NGUYEN-DINH, An, 37520 La Riche (FR)
(74) Representative: Dupuis-Latour, Dominique

(56) References cited:
- EP-A1- 2 594 314
- KR-B1- 101 656 723
- US-A1- 2016 045 724
- US-A1- 2017 151 429
- US-A1- 2019 091 479
- US-B1- 7 771 838
- US-B1- 8 329 314

## Description

### BACKGROUND OF THE INVENTION

### Field of the invention

The invention relates to implantable medical devices, in particular devices of the autonomous implantable capsule type.

Recent advances in miniaturization of active devices and advances in life sciences allow from now on the development of a wide variety of fully autonomous, miniaturized implantable systems, for monitoring, diagnosis or treatment purposes. Such devices implement less invasive implantation procedures, provide more comfort, increased performances, and often open up access to new types of diagnoses and treatments.

A typical although non-limitative example of such autonomous implantable capsules is cardiac implants, which are commonly called "leadless capsules" for distinguishing them from devices or sensors arranged at the distal end of a lead, through the whole length of which run one or several conductors connected to a generator casing which is connected to the opposite, proximal end of the lead.

In the cardiac application case, leadless capsules allow continuously monitoring the cardiac rhythm of the patient and if necessary issuing to the heart electrical pulses for stimulation, resynchronization and/or defibrillation in case of rhythm disorders detected by the capsule.

A cardiac leadless capsule may be an epicardial capsule fixed to the outer wall of the heart or, more often, an endocavitary capsule fixed to the inner wall of a ventricular or atrial cavity, or also fixed to the wall of a vessel near the myocardium.

The invention is nevertheless not limited to a particular type of capsule, or even of leadless capsule, and it is applicable to many other types of implantable medical devices, whether autonomous or not, and whatever the operational purpose thereof, cardiac or other.

### Description of the related art

An implant of the leadless type has generally the cylindrical shape of a capsule so as to be able to be introduced longitudinally through a catheter system passing for example through the patient's venous system. At the end of the cylindrical shape is located a fixation device for anchoring the implant at the desired place for stimulation and detection. A hollow elongated tubular body encloses the different functional elements, electronic circuits and sources of energy.

Such a capsule is described for example in US 2017/0151429 A1 (Regnier), or in WO 2019/001829 A1 and US 2019/091479 A1, both assigned to the present applicant.

For remote exchange of data with other devices, the leadless capsule is provided with a wireless transmitter/receiver circuit and an associated antenna system, coupled to this transmitter/receiver circuit.

Indeed, insofar as the capsule is fully autonomous and is not connected to a remote device by any conductor, the communication with the outside is necessarily operated wirelessly.

It may be in particular a communication with a "telemetry box" at the disposal of a practitioner for configuring, from outside the patient's body, the different operation settings and/or for reading data stored in a memory of the device. It may also be a communication with an implanted or an external box worn by the patient, which plays for example the role of a master device by receiving signals collected by one or several implanted capsules, analyses these signals and optionally sends instructions to such or such capsule for the latter to issue stimulation pulses as needed. The box may also be used as a gateway with the external world to communicate with external peripherals such as programming or remote data transmission devices.

The currently preferred wireless communications technique is the one which uses the components of an electromagnetic wave produced by the transmitter/receiver circuits operating in the radiofrequency (RF) range, typically frequencies of the order of several hundreds of megahertz or a few gigahertz.

The frequencies typically used are those of the band 402-405 MHz called MICS (*Medical Implant Communication System*) band, or 400-407 MHz called MEDS (*Medical External Device Service*) band, or ISM (Industrial, Scientific and Medical) citizens radio bands 433-434 MHz, 863-870 MHz, 902-928 MHz and 2,4 GHz used by medical devices.

The reliability of RF communications with the implant, both for transmission and for reception, as well as the quality of the communication link, are of course essential aspects. The quality of transmission is also an important aspect, because a too high error rate leads to a useless repetition of the messages exchanged, which is harmful as regards the energy autonomy. The arrangement of the transmitter/receiver circuits of the device and of the associated antenna system is hence an important aspect to be considered during the design of such a device.

In another use case, RF circuits may also be used for wireless induction charging (i.e. using mainly the magnetic component of the electromagnetic RF wave) of a power source built in the device, such as a rechargeable battery.

In any case, the metallic nature of the leadless capsule body is however an obstacle to the propagation of the electromagnetic RF wave.

A solution disclosed by the US 2017/0281955 A1 (Maile et al.) consists in providing a system of surface antennas applied on an outer wall of the tubular housing of the leadless device. Admittedly, this technique provides a very good radiation pattern of the system of antennas, but its practical implementation is however difficult. Moreover and above all, it comes up against constraints of biocompatibility of the materials used for the antennas, because the elements of the latter are in permanent contact with the organic fluids, in particular with the blood flow in the case of an implanted autonomous endocardial device. Finally, positioning the different elements of the system of antennas outside the housing implies making feedthroughs for the coupling with the internal circuits, which further complicate the complexity and the final cost of the device. Another solution is disclosed by WO 2004/002572 A1 (Klosterman et al.), in which the RF antenna is housed inside the housing and the latter is made of two parts, one half being formed of a zirconia ceramic tube and another half being formed of a titanium tube. The RF antenna is housed in the ceramic part, which is permeable to RF waves and hence do not disturb the propagation of these waves.

Functionally such a structure is perfectly efficient, but from the technological and industrial point of view its implementation is very complex.

Indeed, obtaining such a heterogeneous housing structure requires assembling two tubular parts of different natures in a perfectly reliable manner, while respecting the severe tightness and biocompatibility constraints specific to medical implants. For that purpose, the above-mentioned document proposes to assemble the titanium tube to the ceramic tube with a metallic intermediate connector inserted into the titanium tube. The zirconia ceramic tube is for its part fit into a counterbore of the connector and sealed to the latter by brazing, with a nickel-titanium alloy as a filler metal, wherein the respective faces of the ceramic tube and of the connector fit into each other provide a self-centering of these elements during the brazing.

This technique of making an interface between the titanium tube and the ceramic tube is relatively complex to implement due to the necessity of providing and adjusting the intermediate connector. Moreover, the nickel of the brazing filler metal is not biocompatible, so that the brazing region must be isolated from the outer environment, in this case by a layer of an isolating material such as parylene, applied to the housing by masking and vapor-phase deposition after previous assembly of both tubes to the connector.

Finally, the fit mounting of the tubes into the connector leads to a structure that is not "isodiametric", i.e. a structure that would have no discontinuity of diameter (inner and/or outer) at the interface between the two metallic and ceramic tubes.

To avoid the use of a non-biocompatible filler metal such as nickel, US 7,771,838 B1 (He et al.) discloses another technique for metal/ceramic assembly applicable to the making of an implantable microstimulator housing provided with an internal RF antenna, which technique teaches the interposition of a titanium-palladium interface layer sandwiched between the ceramic part (for example, made of zirconia) and the titanium part. However, as this technique uses an intermediate layer between the zirconia and the titanium, its industrial implementation is complex. Moreover, biocompatibility of the palladium in the very long run is not as good as that of titanium or zirconia, from which the rest of the envelope housing of the implant is made.

Other prior-art documents disclosing similar implantable medical devices are US 2006/045724 A1 (Lee et al.) and US 8,329,314 B1 (He et al.)

### SUMMARY OF THE INVENTION

The invention is defined in claim 1. Further aspects and preferred embodiments are defined in the appended claims. Aspects, embodiments and examples of the present disclosure which do not fall under the scope of the appended claims do not form part of the invention and are merely provided for illustrative purposes. The object of the invention is a new ceramic/metal composite tube structure for an implantable medical device, in particular for a leadless cardiac capsule, that mitigates the drawbacks of the known structures, in particular the drawbacks exposed hereinabove resulting from a ceramic/metal interface of complex implementation and expensive industrialization.

The invention proposes for that purpose an implantable medical device comprising an elongated composite tubular body including at least one tubular part made of a biocompatible and RF wave permeable dielectric material, and at least one tubular part made of a biocompatible metallic material. A brazing sealing is provided at an interface between the dielectric material tubular part and the metallic material tubular part. At the interface the dielectric material tubular part and the metallic material tubular part have respective planar end faces which are arranged end-to-end against each other without being fit into each other. The interface comprises a planar brazing which seals to each other the two opposite respective planar end faces of the dielectric material tubular part and of the metallic material tubular part.

According to various advantageous subsidiary features:
the interface comprises no overlapping and no fitting of the dielectric material tubular part with the metallic material tubular part;
the elongated tubular body has an isodiametric outer surface, the dielectric material tubular part and the metallic material tubular part having a same outer diameter on either side of the interface;
the elongated tubular body has an isodiametric inner surface, the dielectric material tubular part and the metallic material tubular part having a same inner diameter on either side of the interface;
in this latter case, the device comprises a central tubular support part housed inside the elongated tubular body, the central support tubular part having an outer diameter adjusted to the inner diameter of the dielectric material tubular part and of the metallic material tubular part;
the dielectric material is a ceramic of the group comprising alumina and zirconia, and the metallic material is titanium, in particular an alumina ceramic of purity ≥ 99,5 %;
the brazing is a brazing obtained by gold brazing on a Ti-Nb primer metallization layer deposited on the planar end face of the dielectric material tubular part;
the dielectric material tubular part and the metallic material tubular part extend in the peripheral direction over the whole circumference of the tubular body;
the composite elongated tubular body further houses at least one RF antenna, and the dielectric material tubular part is located axially and circumferentially in alignment with the at least one RF antenna;
the elongated tubular body comprises a central dielectric material tubular element, open at its two ends, and two lateral metallic material tubular elements arranged on either side of the central dielectric material tubular element;
in this latter case, the two lateral metallic material tubular elements are electrically isolated from each other by the central dielectric material tubular element and form between the two distal and proximal ends of the device body a dipole coupled to detection and/or stimulation circuits of the device, for the detection and/or stimulation of an organ with which the device is in contact.

### BRIEF DESCRIPTION OF THE DRAWINGS

The foregoing and other objects, aspects and advantages of the invention will be better understood from the following detailed description of a preferred embodiment of the invention with reference to the appended drawings, in which the same numerals refer to identical or functionally similar features over the different figures.
Figure 1 illustrates medical devices of the leadless capsule type in their environment, with various examples of implantation sites in, on, or near the heart of a patient.
Figure 2 is a longitudinal overall view of a leadless capsule.
Figure 3 shows, as a block diagram, the main internal constitutive elements of the leadless capsule.
Figure 4 is a longitudinal cross-section of the central tubular part of the leadless capsule of Figure 2, showing the mechanical configuration of the different elements located inside the tubular body.
Figure 5 is a cross-sectional view of the tubular body of the capsule of

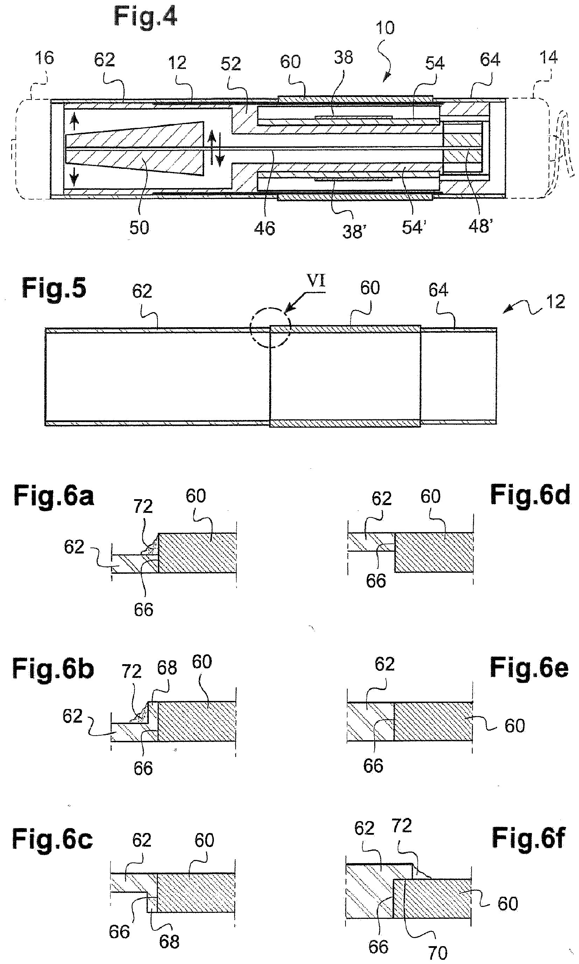

Figure 4, separately shown before the positioning of the internal elements.
Figures 6a to 6f are detailed views, corresponding to mark VI in Figure 5, illustrating various ways for assembling the two constitutive elements of the composite tube of Figure 5.

### DETAILED DESCRIPTION OF

### A PREFERRED EMBODIMENT OF THE INVENTION

An exemplary embodiment of a device implementing the teachings of the invention will now be described, with reference to the drawings.

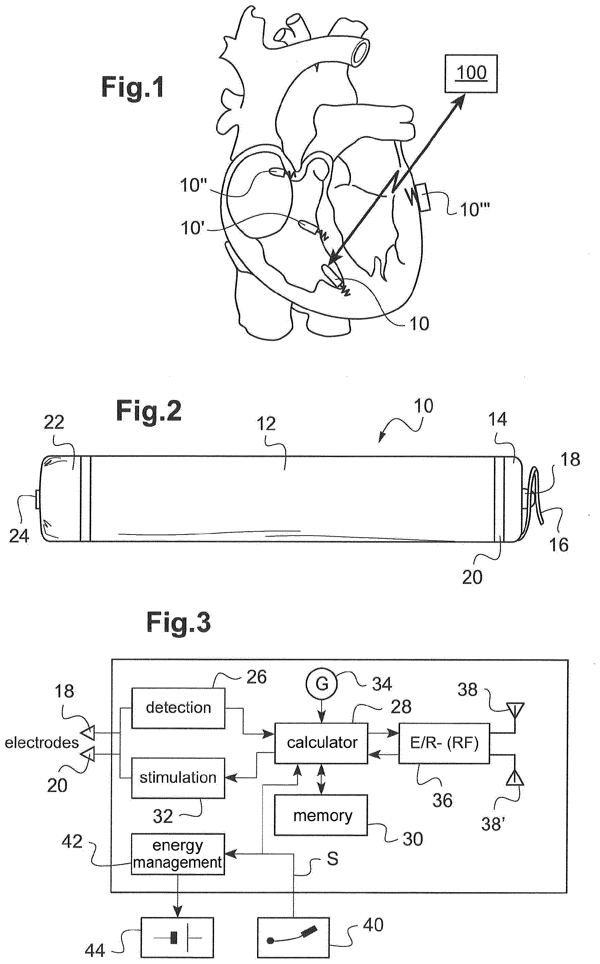

In Figure 1 are shown various possibilities of implantation sites for a device of the leadless type, in a cardiac stimulation application. To this respect, capsule 10 is implanted into a cavity of the myocardium (endocavitary implant), for example at the apex of the right ventricle. As a variant, the capsule may also be implanted on the right interventricular septum, as at 10', or also on an atrial wall, as at 10". The device may also be an epicardial capsule placed on an external region of the myocardium, as at 10"'.

In any case, the leadless capsule is fixed to the cardiac wall by means of a protruding anchoring system penetrating the cardiac tissue for holding it to the implantation site. Other anchoring systems can be used, and do not modify in any way the implementation of the present invention.

The leadless capsules are provided with wireless transmitter/receiver means for the communication with a remote equipment 100, implanted into or external to the patient for the transmission of signals from the capsule 10 to the equipment 100 and, conversely, from the equipment 100 to the capsule 10. In the case several leadless capsules are implanted at the same time, RF communication may also take place directly from one capsule to another capsule, for example between capsules 10 and 10" of Figure 1 in the case of a dual-chamber, atrial and ventricular detection/stimulation system.

Figure 2 is a longitudinal overall view of a leadless capsule 10 in external form of a cylindrical tubular implant body 12 enclosing the various electronic and power supply circuits of the capsule.

The typical dimensions of such a capsule are a diameter of the order of 6 mm and a length of about 25-40 mm.

The elongated tubular body 12 is closed at its distal end by an element 14 carrying a helical screw 16 for anchoring the capsule to a wall of a cardiac cavity, as illustrated hereinabove with respect to Figure 1 (the anchoring mode being of course in no way limitative). A detection/stimulation electrode 18, in contact with the cardiac tissue at the implantation side, operates to collect cardiac depolarization potentials and/or to apply stimulation pulses (in certain embodiments, the function of electrode 18 is performed by the anchoring screw 16, which is accordingly an active, electrically conductive screw connected to the detection/stimulation circuit of the capsule). The electrode 18 in contact with the tissues is generally a cathode, and it is associated with an anode whose function is fulfilled by a second, distinct electrode, most often an annular electrode such as at 20. At the opposite, proximal end, the elongated tubular body is closed by an element 22 having an atraumatic rounded shape and provided with suitable gripping means 24, for the temporary connection of the capsule 10 to a guide-catheter or another accessory usable at the time of implantation or explantation of the capsule.

Figure 3 is a synoptic of the various electric and electronic circuits built in te capsule, shown as functional blocks. These circuits are advantageously made as an ASIC or a combination of ASICs.

Block 26 denotes a heart depolarization wave detection circuit, connected to electrode 18 in contact with the cardiac tissue and to the opposite electrode 20. This detection block 26 comprises filters and means for analog and/or digital processing of the collected signal. The processed signal is applied to the input of a computer 28 associated to a memory 30. The electronic unit also includes a stimulation circuit 32 operating under the control of computer 28 to issue as necessary myocardium stimulation pulses to the electrode system 18, 20. The capsule may also include built-in sensors such as an accelerometer (G-sensor) 34 coupled to the computer 28.

The capsule 10 further includes a radiofrequency (RF) module 36 associated to one or several transmission/reception antennas 38, 38'. In the illustrated example, the RF module is a transmitter/receiver module for wireless communication with a remote equipment 100 (Figure 1) or with other implanted capsules (for example, in the configuration of a dual-chamber ventricular and atrial detection/stimulation system). The RF module, in another case, may be used for other purposes than the communication of data, in particular it may serve to wirelessly couple the capsule to an external device for inductive charging of the battery, i.e. mainly from the magnetic component of the electromagnetic RF wave. The capsule 10 further includes, for the power supply thereof, an energy harvesting module typically comprising an energy harvester 40 comprising an inertial unit that oscillates inside the capsule under the effect of the multiple external stresses to which is subjected the capsule, and performs a function of mechanical-electrical transducer to convert these mechanical stresses into electrical charges, so as to produce a variable electrical signal S that is sent to an energy harvesting circuit 42. Circuit 42 rectifies and regulates the signal S so as to output a stabilized direct voltage or current for powering all the electronic circuits of the capsule, as well as for charging a built-in energy storage component 44, for example a rechargeable battery or a high-performance capacitor.

Figure 4 is a schematic cross-sectional view of the capsule of Figure 2 showing the different elements arranged inside the elongated tubular body 12.

The inertial unit of the energy harvester module, comprising a piezoelectric beam 46 clamped at one of its end 48 and whose opposite, free end, is coupled to an inertial mobile mass 50, is particularly illustrated in this figure. The piezoelectric beam 46 is an elastically deformable flexible beam that constitutes, with the inertial mass 28, a pendular system of the mass-spring type that, due to the inertia of the mass 50, oscillates as soon as the elastic beam 46 is deflected from its stable rest position. The piezoelectric beam 46 further performs a function of mechanical-electrical transducer for converting into electrical charges the mechanical stress that is applied to it when it is bent under the effect of the inertia of the mass 50. These electrical charges are collected by electrodes formed at the surface of the beam 46 to produce the electrical signal S that, after rectification, stabilization and filtering, will power the various electronic circuits of the capsule.

The elongated tubular body 12 further houses an inner part 52 serving both as a support for the piezoelectric beam 46 at its clamping point 48, and as a support for printed circuit boards (PCBs) such as 54 and 54'. Indeed, in order to optimize as far as possible the available space inside the capsule, all built-in electronics are made as electronic circuits advantageously mounted on printed circuit boards 54, 54' located near the inner wall of the elongated tubular body (so as to clear up the central space for the oscillating beam 46), the two circuits 54, 54' being connected to each other by a flexible conductor ribbon.

In addition to the various integrated circuits and discrete components of the electronic unit, the PCBs 54, 54' support the antennas 38, 38', wherein these latter can be made as added discrete components, or as tracks etched on the PCBs.

The presence of two antennas 38, 38' arranged opposite to each other makes it possible to widen the whole radiation diagram of the capsule, making the latter almost omnidirectional, each of the two antennas 38 or 38' covering a substantially hemispherical sector of the radiation diagram. The two-antenna embodiment is however not limitative in any way, and the system may include for example three antennas arranged at 120°, four antennas at 90°, etc., providing an almost omnidirectional radiation pattern.

In order for the RF waves to be able to pass through the body 12, the latter is made as a composite tube with, in alignment with the antennas RF 38, 38', an RF wave permeable part, the rest of the composite tube being made of titanium, i.e. in the areas that do not need to let an RF radiation through.

In Figure 5 is illustrated separately such a composite tube, constituting the elongated tubular body 12 of the capsule 10.

The latter comprises a central part 60, preferably fully tubular, made of an RF wave permeable dielectric material in the region located in alignment with the antennas 38, 38' carried by the PCBs 54, 54'.

On either side of the central tubular part 60 made of a dielectric material, the tube is formed by two metallic tubular elements 62, 64 extending in the regions located away from the antennas 38, 38' and that will hence have no or little incidence on the whole radiation diagram of the system of antennas 38, 38'.

It will be noted that, in this configuration, from the electrical point of view, the two distal 62 and proximal 64 tubular elements are isolated by the central tubular part 60 made of dielectric material, interposed between them. This electrical isolation, which is a direct consequence of the composite design of the tubular body, may be advantageous for example when it is desired to create a dipole between the two distal and proximal ends of the capsule body. Such a dipole may in particular serves for the detection and/or the stimulation of the organ with which it is in contact. The dielectric material of the tubular central part 60 is advantageously a ceramic, for example of the zirconia or alumina type.

Very preferentially, the chosen material is alumina rather than zirconia, although the latter is usually the material the most used in the particular applications considered herein (implants, in particular endocavitary cardiac implants).

Zirconia has indeed excellent mechanical properties, better that alumina, and that is the reason of its preferential choice in the state of the art, as disclosed in particular in above-mentioned WO 2004/002572 A1 and US 7,771,838 B1.

However, very long term strength tests that have been performed have shown a risk of progressive degradation over time of these mechanical properties, due to a possible change of phase, under the only effect of ageing, of a tetragonal crystallographic structure into a monoclinic structure, which may be unstable: this evolution may introduce a progressive loss of the good initial mechanical properties of the material, which, in the worst case, could make it friable.

Due to the compelling need to preserve all the initial properties of the implant, including of course the mechanical robustness of its housing, over the whole expected life duration after implantation (i.e. for at least 10 years), this risk cannot be incurred in any way. That is why, in the case of the composite structure of the invention, the preferred dielectric material is alumina, in particular an alumina having a very high purity grade, higher than or equal to 99,5 %.

The thickness of the dielectric material of the central tubular part 60 is typically 0.1 to 0.6 mm. The shape of the central tubular part 60 is preferably that of a through tube, open at its two ends.

As a variant, the part of the housing that is made of dielectric material could however be made as a blind tube, this dielectric part then continuing up to one of the ends of the envelope housing of the device. For the metallic material of the lateral tubular parts 62, 64, titanium is advantageously chosen. The thickness of the titanium tube in these regions 62, 64 is typically of the order of 0.15 to 0.1 mm.

An essential characteristic common to the dielectric material of the central tubular part 60 and to the metallic material of the lateral tubular parts 62, 64 is that they are perfectly biocompatible and biostable. They hence do not need to be coated with a protective isolating layer, and may be in direct and permanent contact with body fluids, in particular with blood in the case of an implanted endocavitary capsule.

The tube 60 made of dielectric material and the tubes 62, 64 made of metallic material are sealed to each other through a brazing directly performed along the whole periphery of the regions in contact of the tubes respectively butted to each other.

Characteristically of the invention, this brazing is a planar brazing, i.e. the brazing is performed between the two end faces formed by the planar, annular edges of the two cylindrical parts located opposite to each other, the filler metal being brought between the two faces butted to each other. In so far as the two tubes are not fit into each other, nor brought together with interposition of an intermediate part (as in the case of above-mentioned WO 2004/002572 A1) or of an intermediate layer of different nature (as in the case of above-mentioned US 7,771,838 B1), it becomes possible to obtain a brazed, composite tube having in axial direction no discontinuity of its inner surface (i.e. the outer surface of the envelope tube of the device being accordingly uniform and having a constant diameter) and/or of its outer surface (i.e. the inner surface of the composite tube being accordingly uniform and having a constant diameter).

First, an external isodiametry of the composite tube provides an homogeneous and smooth outer surface over the whole length of the housing of the implant, providing the latter with an excellent surface atraumaticity. The metal/ceramic composite outer surface may from then on be kept as such (it will then be in direct contact with the body fluids and tissues), or possibly coated over all or part of its extent with a layer of a suitable coating material, for example parylene, a material used in particular for its hydrophobic and low friction coefficient properties. Second, an internal isodiametry of the composite tube facilitates the mounting of the internal elements of the device, by allowing in particular the use of a multifunctional central part as the one described in copending application USSN 16/393,924 of April 24, 2019, assigned to the present applicant and incorporated herein by way of reference. This application discloses a tubular part made of plastic material, acting in particular as a support for the different components and mechanical and electrical subassemblies of the capsule: these elements are beforehand mounted and interconnected on the tubular support part, then the latter, so equipped, is inserted into the envelope tube of the capsule through an end opening, by simple axial translation over the whole length of the tube. The particular geometry of the support part and a suitable choice of the plastic material make it possible to provide self-centering during this operation and, at the end of the displacement, securing of the support part to the envelope tube with a tight fit.

The implementation of this specific support part and of the simplified assembly process thus allowed requires to be able to introduce it and to push it over the whole length of the envelope tube, which precisely is possible thanks to the internal isodiametry of the tube.

The brazing technique used is very preferentially a technique that only implements fully biocompatible materials. For the assembly of an alumina tube to a titanium tube, the process may for example comprise two successive steps, with a deposition of a metallization on the surface to be brazed on the alumina side by a metallic layer such as a titanium/niobium alloy, followed with a brazing of the two parts to each other with molten gold as a filler metal.

This way to proceed avoids the drawbacks of the technique disclosed by the above-mentioned WO 2004/002572 A1, in particular it avoids the use of a non-biocompatible metal (nickel) in the nickel-titanium alloy used for an active brazing, whereby the nickel-titanium alloy plays a double role of a primer metallization on the zirconia and of a filler metal between the two parts to be brazed.

The implemented brazing process parameters (temperature, operating mode, etc.) are *per se* conventional, they only need to be adapted to the geometry (planar and annular surfaces) and dimensions of the brazing interface. The techniques used and the respective advantages will depend in particular on the thicknesses of the tubes and on their respective conformation. In this respect, it will be noted that, due to the planar brazing between the butted surfaces of the dielectric tube 60 and of the metallic tube 62, the wall thickness of the tubes has a direct incidence on the brazing surface. Concretely, for the assembly to keep satisfying mechanical properties, it seems desirable to limit this thickness to a minimum wall value of 0.4 mm.

Figures 6a to 6f illustrate various tube configurations at the interface between the dielectric material tube 60 and the metallic material tube 62.

Figures 6a, 6b, 6e and 6f illustrate an example of assembly with an internal isodiametry, and Figures 6c, 6d and 6e an example of assembly with an external isodiametry.

To increase the surface of contact at the interface 66 between the two metallic and ceramic tubes, it may be advantageous to machine the metallic part 62 so as to form a flange 68 (Figures 6b and 6c) allowing making up for the thickness difference between the thinner, metallic tube, and the thicker, ceramic tube. In a reverse configuration, it is possible to provide a thicker metallic tube, machined with a counterbore 70 into which the ceramic tube 60 will be housed (Figure 6f).

Advantageously, in the case of an external discontinuity between the two tubes (Figures 6a, 6b and 6f), a meniscus of glue 72 can be added to eliminate the sharp edge and increase the atraumaticity of the outer surfaces of the capsule.

In all these configurations, the brazing is performed at the planar interface 66 between the respectively butted contact regions. The presence of a flange or a shoulder is only intended essentially to make up for the different thicknesses or to facilitate a respective centering of the two tubes but essentially it does not participate to the brazing process.

## Claims

1. An implantable medical device (10) comprising an elongated composite tubular body (12) including at least one tubular part made of a biocompatible and RF wave permeable dielectric material (60), and at least one tubular part made of a biocompatible metallic material (62, 64), wherein a brazing sealing is provided at an interface (66) between the dielectric material tubular part (60) and the metallic material tubular part (62, 64),
**characterized in that** at the interface (66) the dielectric material tubular part (60) and the metallic material tubular part (62, 64) have respective planar end faces which are arranged end-to-end against each other without being fit into each other,
and **in that** the brazing sealing is a planar brazing which seals to each other the two opposite respective planar end faces of the dielectric material tubular part (60) and of the metallic material tubular part (62, 64).

2. The device of claim 1, wherein the interface (66) comprises no overlapping and no fitting of the dielectric material tubular part (60) with the metallic material tubular part (62, 64).

3. The device of claim 1, wherein the elongated tubular body (12) has an isodiametric outer surface, the dielectric material tubular part (60) and the metallic material tubular part (62, 64) having a same outer diameter on either side of the interface (66).

4. The device of claim 1, wherein the elongated tubular body (12) has an isodiametric inner surface, the dielectric material tubular part (60) and the metallic material tubular part (62, 64) having a same inner diameter on either side of the interface (66).

5. The device of claim 4, comprising a central tubular support part (52) housed inside the elongated tubular body (12), the central support tubular part (52) having an outer diameter adjusted to the inner diameter of the dielectric material tubular part (60) and of the metallic material tubular part (62, 64).

6. The device of claim 1, wherein the dielectric material is a ceramic of the group comprising alumina and zirconia, and the metallic material is titanium.

7. The device of claim 6, wherein the dielectric material is an alumina ceramic of purity ≥ 99,5 %.

8. The device of claim 6, wherein the brazing is a brazing obtained by gold brazing on a Ti-Nb primer metallization layer deposited on the planar end face of the tubular part made of dielectric material.

9. The device of claim 1, wherein the dielectric material tubular part (60) and the metallic material tubular part (62, 64) extend in the peripheral direction over the whole circumference of the tubular body (12).

10. The device of claim 1, wherein the composite elongated tubular body (12) further houses at least one RF antenna (38, 38'),
and wherein the dielectric material tubular part (60) is located axially and circumferentially in alignment with the at least one RF antenna (38, 38').

11. The device of claim 1, wherein the elongated tubular body (12) comprises a central dielectric material tubular element (60), open at its two ends, and two lateral metallic material tubular elements (62, 64) arranged on either side of the central dielectric material tubular element (60).

12. The device of claim 11, wherein the two lateral metallic material tubular elements (62, 64) are electrically isolated from each other by the central dielectric material tubular element (60) and form between the two distal and proximal ends of the device body a dipole coupled to detection (26) and/or stimulation (32) circuits of the device, for the detection and/or stimulation of an organ with which the device is in contact.

## Patentansprüche

1. Implantierbare medizinische Vorrichtung (10), umfassend einen länglichen röhrenförmigen Verbundkörper (12), der mindestens einen röhrenförmigen Abschnitt, der aus einem bioverträglichen und für Hochfrequenzwellen durchlässigen dielektrischen Material (60) hergestellt ist, und mindestens einen röhrenförmigen Abschnitt umfasst, der aus einem bioverträglichen metallischen Material (62, 64) hergestellt ist,
wobei an einer Grenzfläche (66) zwischen dem röhrenförmigen Abschnitt (60) aus dielektrischem Material und dem röhrenförmigen Abschnitt (62, 64) aus metallischem Material eine abdichtende Hartlotverbindung bereitgestellt ist,
**dadurch gekennzeichnet, dass** der röhrenförmige Abschnitt (60) aus dielektrischem Material und der röhrenförmige Abschnitt (62, 64) aus metallischem Material an der Grenzfläche (66) jeweils ebene endständige Flächen aufweisen, die derart angeordnet sind, dass ihre Enden ohne formschlüssige Verbindung aneinanderstoßen,
und dadurch, dass es sich bei der abdichtenden Hartlotverbindung um eine ebene Hartlotverbindung handelt, welche die beiden gegenüberliegenden jeweils ebenen endständigen Flächen des röhrenförmigen Abschnitts (60) aus dielektrischem Material und des röhrenförmigen Abschnitts (62, 64) aus metallischem Material abdichtend miteinander verbindet.

2. Vorrichtung nach Anspruch 1, wobei die Grenzfläche (66) keinerlei Überlappung und keinerlei Formschluss des röhrenförmigen Abschnitts (60) aus dielektrischem Material mit dem röhrenförmigen Abschnitt (62, 64) aus metallischem Material umfasst.

3. Vorrichtung nach Anspruch 1, wobei der längliche röhrenförmige Körper (12) eine isodiametrische Außenfläche aufweist, wobei der röhrenförmige Abschnitt (60) aus dielektrischem Material und der röhrenförmige Abschnitt (62, 64) aus metallischem Material beiderseits der Grenzfläche (66) denselben Außendurchmesser aufweisen.

4. Vorrichtung nach Anspruch 1, wobei der längliche röhrenförmige Körper (12) eine isodiametrische Innenfläche aufweist, wobei der röhrenförmige Abschnitt (60) aus dielektrischem Material und der röhrenförmige Abschnitt (62, 64) aus metallischem Material beiderseits der Grenzfläche (66) denselben Innendurchmesser aufweisen.

5. Vorrichtung nach Anspruch 4, umfassend einen mittigen röhrenförmigen Trägerabschnitt (52), der in dem länglichen röhrenförmigen Körper (12) aufgenommen ist, wobei der mittige röhrenförmige Trägerabschnitt (52) einen Außendurchmesser aufweist, der an den Innendurchmesser des röhrenförmigen Abschnitts (60) aus dielektrischem Material und des röhrenförmigen Abschnitts (62, 64) aus metallischem Material angepasst ist.

6. Vorrichtung nach Anspruch 1, wobei es sich bei dem dielektrischen Material um einen keramischen Werkstoff aus der Gruppe handelt, umfassend Aluminiumoxid und Zirconiumdioxid, und es sich bei dem metallischen Material um Titan handelt.

7. Vorrichtung nach Anspruch 6, wobei es sich bei dem dielektrischen Material um einen keramischen Aluminiumoxidwerkstoff mit einem Reinheitsgrad von ≥ 99,5 % handelt.

8. Vorrichtung nach Anspruch 6, wobei es sich bei der Hartlotverbindung um eine Hartlotverbindung handelt, die erhalten wurde, indem ein Goldhartlot auf eine Metallisierungsschicht aus einer Ti-Nb-Grundierung aufgebracht wird, die auf der ebenen endständigen Fläche desjenigen röhrenförmigen Abschnitts abgeschieden wurde, der aus dielektrischem Material hergestellt ist.

9. Vorrichtung nach Anspruch 1, wobei der röhrenförmigen Abschnitt (60) aus dielektrischem Material und der röhrenförmige Abschnitt (62, 64) aus metallischem Material sich in randständiger Richtung über den gesamten Umfang des röhrenförmigen Körpers (12) erstrecken.

10. Vorrichtung nach Anspruch 1, wobei in dem länglichen röhrenförmigen Verbundkörper (12) des Weiteren mindestens eine HF-Antenne (38, 38') aufgenommen ist
und wobei der röhrenförmige Abschnitt (60) aus dielektrischem Material in axialer und umfangsbezogener Hinsicht auf die mindestens eine HF-Antenne (38, 38') ausgerichtet ist.

11. Vorrichtung nach Anspruch 1, wobei der längliche röhrenförmige Körper (12) ein mittiges röhrenförmiges Element (60) aus dielektrischem Material, das an seinen beiden Enden offen ist, und zwei seitliche röhrenförmige Elemente (62, 64) aus metallischem Material umfasst, die beiderseits des mittigen röhrenförmigen Elements (60) aus dielektrischem Material angeordnet sind.

12. Vorrichtung nach Anspruch 11, wobei die beiden seitlichen röhrenförmigen Elemente (62, 64) aus metallischem Material durch das mittige röhrenförmige Element (60) aus dielektrischem Material elektrisch voneinander isoliert sind und zwischen den beiden distalen und proximalen Enden des Vorrichtungskörpers einen Dipol bilden, der an Schaltkreise zur Erfassung (26) und/oder Stimulierung (32) der Vorrichtung gekoppelt ist, um ein Organ, mit dem die Vorrichtung in Kontakt ist, zu erfassen und/oder zu stimulieren.

## Revendications

1. Un dispositif médical implantable (10) comprenant un corps tubulaire allongé composite (12) comprenant au moins une partie tubulaire en un matériau diélectrique biocompatible et perméable aux ondes RF (60), et au moins une partie tubulaire en un matériau métallique biocompatible (62, 64),
dans lequel il est prévu un scellement brasé à une interface (66) entre la partie tubulaire en matériau diélectrique (60) et la partie tubulaire en matériau métallique (62, 64),
**caractérisé en ce qu'**à l'interface (66) la partie tubulaire en matériau diélectrique (60) et la partie tubulaire en matériau métallique (62, 64) présentent des faces planes d'extrémité respectives qui sont agencées bout à bout l'une contre l'autre sans emboitement l'une dans l'autre,
et **en ce que** le scellement brasé est une brasure plane qui scelle l'une à l'autre les deux faces planes d'extrémité respectives en vis-à-vis de la partie tubulaire en matériau diélectrique (60) et de la partie tubulaire en matériau métallique (62, 64).

2. Le dispositif de la revendication 1, dans lequel l'interface (66) ne comprend ni recouvrement ni emboitement de la partie tubulaire en matériau diélectrique (60) avec la partie tubulaire en matériau métallique (62, 64).

3. Le dispositif de la revendication 1, dans lequel le corps tubulaire allongé (12) présente une surface externe isodiamétrique, la partie tubulaire en matériau diélectrique (60) et la partie tubulaire en matériau métallique (62, 64) ayant un diamètre externe identique de part et d'autre de l'interface (66).

4. Le dispositif de la revendication 1, dans lequel le corps tubulaire allongé (12) présente une surface interne isodiamétrique, la partie tubulaire en matériau diélectrique (60) et la partie tubulaire en matériau métallique (62, 64) ayant un diamètre interne identique de part et d'autre de l'interface (66).

5. Le dispositif de la revendication 4, comprenant une pièce support tubulaire centrale (52) logée à l'intérieur du corps tubulaire allongé (12), la pièce tubulaire support centrale (52) ayant un diamètre externe ajusté au diamètre interne de la partie tubulaire en matériau diélectrique (60) et de la partie tubulaire en matériau métallique (62, 64).

6. Le dispositif de la revendication 1, dans lequel le matériau diélectrique est une céramique du groupe comprenant l'alumine et la zircone, et le matériau métallique est le titane.

7. Le dispositif de la revendication 6, dans lequel le matériau diélectrique est une céramique d'alumine de pureté ≥ 99,5 %.

8. Le dispositif de la revendication 6, dans lequel la brasure est une brasure obtenue par brasage à l'or sur une couche de métallisation d'accrochage Ti-Nb déposée sur la face plane d'extrémité de la partie tubulaire en matériau diélectrique.

9. Le dispositif de la revendication 1, dans lequel la partie tubulaire en matériau diélectrique (60) et la partie tubulaire en matériau métallique (62, 64) s'étendent en direction périphérique sur la totalité de la circonférence du corps tubulaire (12).

10. Le dispositif de la revendication 1, dans lequel le corps tubulaire allongé composite (12) loge en outre au moins une antenne RF (38, 38'), et dans lequel la partie tubulaire en matériau diélectrique (60) est située axialement et périphériquement au droit de la au moins une antenne RF (38, 38').

11. Le dispositif de la revendication 1, dans lequel le corps tubulaire allongé (12) comprend un élément tubulaire central en matériau diélectrique (60) ouvert à ses deux extrémités, et deux éléments tubulaires latéraux en matériau métallique (62, 64) agencés de part et d'autre de l'élément tubulaire central en matériau diélectrique (60).

12. Le dispositif de la revendication 11, dans lequel les deux éléments tubulaires latéraux en matériau métallique (62, 64) sont électriquement isolés entre eux par l'élément tubulaire central en matériau diélectrique (60) et forment entre les deux extrémités distale et proximale du corps du dispositif un dipôle couplé à des circuits de détection (26) et/ou de stimulation (32) du dispositif, pour la détection et/ou la stimulation d'un organe avec lequel le dispositif est en contact.
